# DEMANDE DE BREVET EUROPEEN

(11) **EP 3 447 489 A1**
(43) Date de publication de la demande: **27.02.2019**
(21) Numéro de dépôt: 18185718.6
(22) Date de dépôt: 26.07.2018
(51) Int. Cl.: G01N 33/12, A22B 5/00

(54) **INSTALLATION ET PROCÉDÉ D'AIDE À LA DÉTECTION DE CARCASSES ODORANTES**

(30) Priorité: 23.08.2017 FR 1757827
(71) Demandeur: Cooperl Innovation SAS, 22400 Lamballe (FR)
(72) Inventeur: LACOSTE, Anne, 35000 RENNES (FR); MELI FOAGUAM, Cyrille, 35000 RENNES (FR)
(74) Mandataire: Regimbeau

(57) **Abrégé**

L'invention se rapporte notamment à une installation (I) d'aide à la détection de carcasses (C) odorantes d'animaux, caractérisée par le fait qu'elle comporte :
au moins un support d'information portant une information relative au moins au sexe dudit animal ;
au moins un outil (2) de lecture de ladite information ;
au moins une sonde (3) de lecture, reliée à un spectromètre infrarouge, pour mesurer le spectre d'absorption infrarouge desdites carcasses (C) ;
au moins un équipement informatique (5) qui enregistre chaque spectre mesuré ;
et qui est configuré pour :
- associer ladite information relative au sexe, lue par ledit outil de lecture (2) avec le spectre infrarouge mesuré associé ;
- comparer ce spectre infrarouge aux spectres de la base de données ;
- si ce spectre n'est pas considéré comme un spectre aberrant, affecter à ladite carcasse ladite note d'odeur associée ;
- visualiser la note obtenue.

## Description

### DOMAINE DE L'INVENTION

La présente invention se rapporte à une installation et à un procédé d'aide à la détection de carcasses odorantes d'animaux abattus précédemment.

### ARRIERE PLAN TECHNOLOGIQUE DE L'INVENTION

La pratique de la castration chirurgicale des porcs est régulièrement remise en cause par les associations de protection animale et de consommateurs, pour des raisons de bien-être animal.

Une déclaration signée en 2010 à Bruxelles par les principaux pays producteurs prévoit de mettre fin à la pratique de la castration à vif des porcelets d'ici 2018.

Ainsi en Europe, environ 28 % des éleveurs de porcs ne castrent plus les porcs, avec un fort développement en Hollande, Allemagne et France.

L'absence de castration des porcs entraîne un développement d'odeurs désagréables sur la viande, qualifiées d'odeurs sexuelles. Elles se manifestent fréquemment lors de la cuisson des viandes de porcs non castrés. Aux stades usuels d'abattage, les porcs non castrés ne présentent pas tous ces odeurs. On estime que seules 3 à 5% des carcasses de porcs non castrés sont détectées comme odorantes.

Toutefois, de nombreuses recherches sont actuellement en cours dans les élevages et les centres de recherches sur les plans génétique, zootechnique et immunitaire, afin de limiter ou réduire les risques d'apparition d'odeurs désagréables dans la production des porcs.

Au sein des abattoirs, les recherches se focalisent essentiellement sur la détection de carcasses odorantes issues de porcs mâles entiers (c'est à dire non castrés) afin de garantir la commercialisation de la viande de porcs, sans risque d'odeurs pour l'ensemble de la filière.

Les résultats d'une enquête européenne (réalisée en 2015) présentant l'état de l'art concernant la détection des carcasses odorantes dans de nombreux abattoirs européens, montre que seulement deux types de méthodes de détection sont en place dans les abattoirs traitant des mâles entiers.

La méthode sensorielle du « nez humain » est la plus répandue (90% des abattoirs traitant des mâles entiers la pratiquent) et s'impose. Toutefois, cette méthode, qui fait appel aux sens de l'homme pour détecter les odeurs de carcasses, présente certains inconvénients (entrainement et subjectivité des opérateurs qualifiés, chauffage préalable, saturation olfactive), etc.).

Actuellement, des recherches sont en cours dans les principaux pays producteurs de mâles entiers pour développer des méthodes instrumentales dont les caractéristiques et performances permettront une utilisation dans des conditions industrielles.

C'est d'ailleurs un souhait de l'Union Européenne ("EC Workshop on piglet castration 2010") pour harmoniser, voire qualifier les méthodes retenues avec des méthodes de référence.

L'odeur de verrat (ou "odeur sexuelle") présente chez les porcs mâles entiers découle principalement d'une accumulation dans les tissus adipeux (graisse) de plusieurs composés, dont les principaux sont l'androstérone, l'indole et le scatol.

Le pourcentage de porcs mâles odorants est faible mais nécessite de qualifier l'ensemble des carcasses de mâles entiers et, de ce fait, de procéder à un repérage au préalable du sexe de l'animal.

Une méthode instrumentale de détection et de tri des carcasses odorantes développée à ce jour est celle connue sous le nom de "Nez Electronique" .

Elle présente de nombreux inconvénients car elle nécessite le captage de l'odeur par chauffage du gras dans une chambre d'ionisation, pour permettre une analyse par chromatographie à partir des spectres des principales molécules recherchées.

Même si la partie analyse peut être miniaturisée, reste l'étape de prélèvement d'échantillon de gras et de capture des composés volatiles, ce qui rend actuellement son usage en site industriel impossible.

Cette méthode étant incompatible avec la détection massive et rapide des carcasses odorantes, se sont développées, dans les outils industriels, des méthodes dites "Nez Humains", basées sur la sélection et l'entraînement de jury expert pour la détection et qualification de ces odeurs (Matmur et al., 2012).

La méthode sensorielle du « nez humain » (mise en place au stade industriel par la société néerlandaise VION en 2011), qui est la plus répandue aujourd'hui dans les abattoirs, présente aussi quelques inconvénients.

En général, elle nécessite la mise en oeuvre de deux opérations, à savoir le chauffage du gras pour révéler les composés odorants, puis la qualification de l'odeur. Ce poste nécessite donc, selon la taille et la cadence de la chaîne de l'abattoir, la présence de deux opérateurs, qui doivent alterner les opérations toutes les 30 minutes afin d'éviter de saturer leurs capacités olfactives.

De plus, pour apprécier l'odeur, l'opérateur doit chauffer le gras en utilisant de l'air chaud (décapeur thermique), une flamme (chalumeau) ou par contact avec une résistance électrique (fer à souder). On comprend aisément que cela peut occasionner des risques de brûlures chez l'opérateur en cas de mauvaise manipulation.

Avec les cadences élevées des lignes d'abattage, l'opérateur n'a pas toujours le temps d'apprécier l'odeur de toutes les carcasses (l'opérateur ayant environ cinq à six secondes pour attribuer une note d'odeur à chaque carcasse). En cas de non détection de l'odeur sur la ligne d'abattage, les carcasses doivent être alors soumises à une évaluation d'odeur ultérieure, avant commercialisation.

Les opérateurs doivent être sélectionnés et entrainés pour pouvoir détecter des odeurs de verrats. Mais toutes les personnes ne sont pas sensibles aux odeurs de scatol et d'androsténone. Et il est parfois difficile de trouver, parmi le personnel, des personnes naturellement très sensibles à ces deux molécules.

Enfin, outre leur entrainement et leur qualification, les opérateurs doivent être calés sur le même niveau de sensibilité afin de rendre cette opération de contrôle équitable et non subjectif.

La qualification des opérateurs experts se fait sur leurs aptitude en termes de sensibilité et de spécificité pour détecter les deux molécules majoritairement à l'origine des odeurs cibles : le scatole et l'androsténone. Ces opérateurs sont entrainés et jugés par une mise en situation à partir d'échantillons qualifiés sur des taux de ces deux molécules obtenus par analyse chimique. Si cette méthode de qualification permet de bien mesurer leur sensibilité pour chacune des molécules, elle ne permet pas la prise en compte des interactions entre molécules olfactives.

Un autre état de la technique en la matière est décrit dans le document DE 10 204 117572. On y décrit une installation et un procédé de détection et de tri de carcasses odorantes.

Cette technique souffre de plusieurs inconvénients.

Ainsi, on décrit dans ce document le fait que la décision du caractère odorant se fait par comparaison avec les seuils de présence de molécules selon une analyse chimique, exclusivement sur les molécules scatol, androstérone et indole.

Cela signifie que le caractère odorant est lié à ces molécules ou à des seuils par molécule, pour lesquels on considère que la viande est odorante.

Les trois molécules précitées font "consensus" pour les seuils odorant/non odorant, mais ces seuils sont dépendants des individus puisque tous les consommateurs n'ont pas la même sensibilité pour chacun de ces trois composés.

De plus, même si les trois composés ci-dessus sont majoritairement impliqués pour caractériser l'odeur recherchée, il en existe une dizaine de plus, certes moins significatifs, mais qui ne sont pas pris en compte.

Par ailleurs, la méthode décrite ne tient pas compte des interactions qui peuvent exister entre les molécules.

Toujours dans ce document, le clivage odorant/non odorant se fait par enregistrement d'un spectre dans l'infra-rouge. Mais rien n'est prévu lorsqu'un spectre est mal enregistré, pour une raison quelconque.

Enfin, pour la prise de mesure spectrale, on fait usage d'une sonde que l'on enfonce dans la carcasse, sur une profondeur de 15 mm.

Or, les composés olfactifs sont des aldéhydes qui sont liposolubles. C'est pourquoi ils sont majoritairement stockés dans les tissus adipeux, bien plus que dans la couenne et les tissus maigres.

La profondeur indiquée ci-dessus correspond à une zone très hétérogène en limite de couenne. C'est pourquoi la méthode décrite ci-dessus ne peut pas fonctionner correctement avec la fiabilité attendue.

La présente invention vise à répondre aux difficultés énumérées ci-dessus. Plus précisément, elle vise à fournir des moyens et un procédé qui, tout en se basant sur la détection de l'odeur par le nez humain, permet de s'en affranchir et de rationnaliser la détection de l'odeur des carcasses.

### RESUME DE L'INVENTION

Ainsi, un premier aspect de la présente invention se rapporte à une installation d'aide à la détection de carcasses odorantes d'animaux abattus précédemment, caractérisée par le fait qu'elle comporte :
- au moins un support d'information, ce support d'information portant, pour chaque carcasse d'animal, une information relative au moins au sexe dudit animal ;
- au moins un outil de lecture et d'enregistrement de ladite information ;
- au moins une sonde de lecture, reliée à un spectromètre infrarouge, ce dernier étant agencé pour mesurer le spectre d'absorption infrarouge desdites carcasses ;
au moins un équipement informatique qui enregistre chaque spectre mesuré , équipement incorporant des algorithmes obtenus suite à une phase d'étalonnage avec une base de données contenant un nombre prédéterminé de "spectres de référence", c'est à dire de spectres d'absorption infra-rouge de carcasses associés à des notes d'odeur, ainsi qu'un certain nombre de "spectres aberrants", à savoir des spectres qui ne respectent pas des paramètres prédéfinis, lesdites notes d'odeur ayant été attribuées préalablement par des opérateurs après détection par le nez humain,
cet équipement informatique étant configuré pour :
- en premier lieu, pour chaque carcasse testée et seulement dans l'hypothèse où la carcasse testée provient d'un animal de sexe masculin, associer ladite information relative au sexe, lue par ledit outil de lecture avec le spectre infrarouge mesuré associé ;
- en deuxième lieu, comparer ce spectre infrarouge mesuré aux spectres de la base de données ;
- en troisième lieu et seulement si le spectre de la base de données le plus proche n'est pas considéré comme un spectre aberrant, affecter à ladite carcasse ladite note d'odeur associée ;
- visualiser la note obtenue en troisième lieu.

Selon d'autres caractéristiques non limitatives et avantageuses de cette installation :
- ledit support d'information porte également une information relative au numéro de tuerie de l'animal ;
- ladite sonde est du type « par pénétration » ou du type « par contact », c'est à dire qu'elle est configurée pour réaliser une lecture par pénétration dans la carcasse, respectivement par contact avec la carcasse ;
- elle comprend des algorithmes configurés pour associer, à ladite carcasse, ladite note suivant le type de sonde ;
- lesdits spectres sont des spectres d'absorption dans le proche infrarouge ;
- ledit équipement informatique est également configuré pour, en quatrième lieu, trier lesdites carcasses en fonction de la note qui leur a été allouée ;
- ledit équipement informatique comprend au moins un algorithme mathématique de prédiction de la note d'odeur ;

La présente invention concerne aussi un procédé qui fait usage de l'installation ci-dessus.

Il est remarquable notamment en ce que qu'il comporte au moins les étapes suivantes :
a/ lecture de ladite information relative au moins au sexe ;
b/ positionnement de ladite sonde relativement à ladite carcasse et activation de celle-ci pour mesurer ledit spectre ;
c/ vérification de la qualité du spectre mesuré, c'est à dire détermination de l'appartenance ou non aux spectres aberrants, avant de lui attribuer une note d'odeur obtenue ;
d/ visualisation de la note obtenue, en troisième lieu, par ledit équipement informatique ;
e/ affectation à ladite carcasse de ladite note.

Selon des caractéristiques avantageuses de ce procédé :
- lorsque la sonde est une sonde de contact, on la positionne en surface de ladite carcasse, préférentiellement sur la partie découverte de l'échine ;
- lorsque la sonde est une sonde de pénétration, on la positionne à une profondeur d'environ 5 cm par rapport à la surface de la carcasse, préférentiellement dans la zone de l'échine.

### BREVE DESCRIPTION DES DESSINS

D'autres caractéristiques et avantages de l'invention apparaîtront à la lecture de la description suivante d'un mode de réalisation préféré de l'invention. Cette description est faite en référence aux dessins annexés dans lesquels :
- la figure 1 est une vue très schématique d'un exemple de réalisation d'une installation conforme à la présente invention ;
- la figure 2 est un organigramme permettant de comprendre le processus mis en oeuvre à l'aide de l'installation de la figure 1.

### DESCRIPTION DETAILLEE DE L'INVENTION

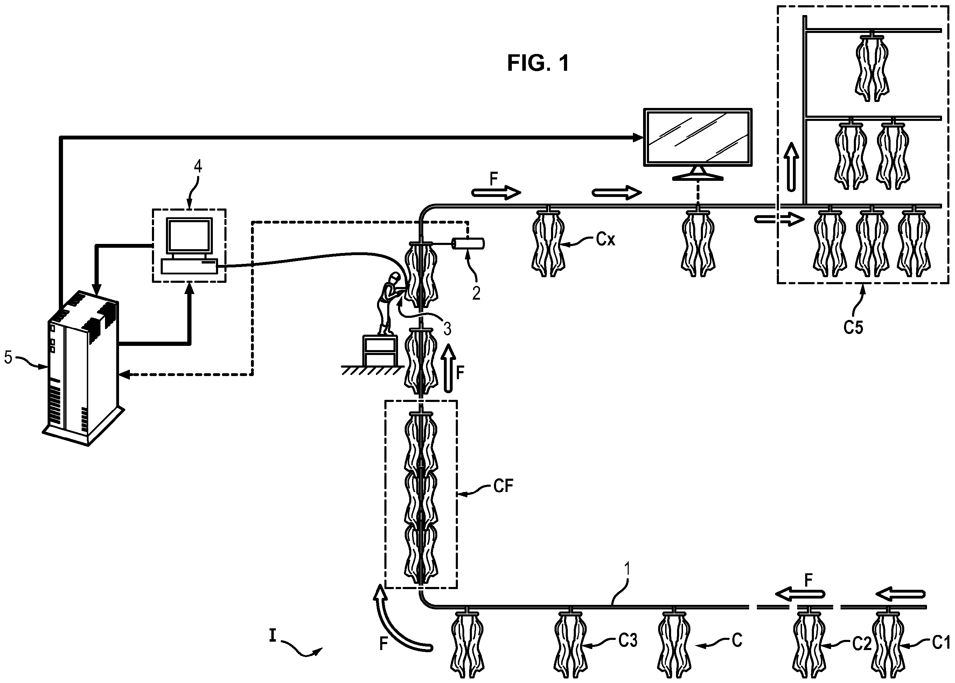

Dans l'exemple illustré à la figure 1 annexée, l'installation I d'aide à la détection de carcasses odorantes d'animaux abattus comporte au moins un convoyeur aérien 1 qui est configuré pour acheminer progressivement, dans le sens des flèches F, des carcasses C d'animaux, et tout particulièrement de porcs.

Dans le présent mode de réalisation, ces carcasses sont référencées C₁, C₂, C₃...Cₓ. Il s'agit préférentiellement de carcasses entières, c'est à dire sur lequel aucune pièce de viande n'a déjà été prélevée. Toutefois, on ne peut exclure le cas dans lequel ces carcasses ne soient pas entières.

Le convoyeur 1 peut être configuré pour être déplacé automatiquement, selon un programme qui tient compte de l'avancée du travail des opérateurs le long de celui-ci.

L'installation I comporte par ailleurs un support d'information non représenté ici.

Dans ce mode de réalisation, ce support est constitué par de carcasse C elle-même et l'information qu'elle comporte est relative au sexe de l'animal correspondant à la carcasse.

On peut également avoir affaire à une information caractéristique du temps écoulé entre l'abattage de l'animal correspondant et l'arrivée de la carcasse C au sein de l'installation I. Cette information correspond par exemple au quantième de la date d'abattage (ou de tuerie). Elle peut comprendre également un numéro de tuerie spécifique à une session particulière d'abattage.

Dans des exemples non représentés, l'information précitée pourrait être portée par un support distinct de la carcasse C. Il pourrait s'agir par exemple d'une étiquette collée sur la carcasse C ou fixée à celle-ci par des moyens connus.

Toutefois, selon l'exemple présenté ici, l'information est portée de manière sûre par la carcasse C quoi qu'il lui arrive, puisque cette information est directement tatouée ou imprimée sur la peau de l'animal.

On peut envisager que cette information ait la forme d'au moins un code-barres d'au moins un QR-Code ou d'au moins deux caractères alphanumériques ou encore d'une combinaison deux de ceux-ci.

Toujours en référence à la figure 1, l'installation comporte, le cas échéant, une chambre froide CF au sein de laquelle les carcasses C transitent momentanément.

Elle comprend également une cellule 2 de détection de l'arrivée d'une carcasse C à un poste de détection d'odeur. A cette fin, la cellule 2 émet un rayonnement du genre laser, et cette cellule constitue un outil de lecture et d'enregistrement de l'information précitée relative, au moins, au sexe de l'animal.

De manière particulièrement avantageuse, l'appartenance de la carcasse C à un des deux sexes aura été faite par un opérateur avant l'entrée dans la chambre froide CF et saisie sur un ordinateur.

Egalement de manière préférée, la carcasse C, via la cellule 2 est repérée par son numéro de tuerie. Ce faisant, l'information relative au sexe est renvoyée à l'opérateur (via un écran, un voyant lumineux, etc.), de manière à lui donner l'ordre de procéder à une mesure d'odeur si le sexe est masculin ou à laisser passer la carcasse sans détection si le sexe est féminin.

Lorsque cette lecture est faite, l'enregistrement correspondant est communiqué à un équipement informatique comprenant un serveur informatique 5 qui stocke l'information. Cette communication entre la cellule 2 et le serveur 5 peut se faire par liaison filaire, par Wi-Fi, ou par Internet.

A titre indicatif, le serveur utilisé est par exemple celui connu sous la référence AS/400 de la société IBM.

L'installation comporte par ailleurs, au moins une sonde de lecture 3 qui n'a pas été représentée sur les figures annexées dans un simple souci de simplification.

Elle comporte avantageusement un corps en plastique qui peut faire office de poignée de manipulation, corps qui est muni d'un bouton d'activation de la sonde.

Le corps présente un canal longitudinal qui accueille un câble de fibres optiques. Dans le prolongement du corps s'étend une tige à l'extrémité libre de laquelle affleure le câble de fibres optiques précité. Cette tige présente préférentiellement un repère qui permet à un opérateur de visualiser la profondeur d'enfoncement de la tige dans la carcasse C.

Dans l'hypothèse où cette sonde est non pas une sonde de contact mais de pénétration, l'extrémité de la tige peut être prévue en pointe.

Le câble de fibres optiques est relié à un spectromètre conformé pour enregistrer un spectre d'absorption dans le proche infrarouge. A titre indicatif, ce spectromètre est par exemple celui commercialisé par ASD - Analytical Spectral Device Incorporated ou par BONSAI ADVANCED TECHNOLOGIES.

De plus, l'installation comporte, en plus du serveur 5, un ordinateur 4 qui fait partie intégrante de l'équipement informatique précité.

Cet équipement informatique enregistre chaque spectre mesuré avec l'installation de l'invention. Dans cet équipement informatiques sont chargés des algorithmes obtenus suite à une phase d'étalonnage avec une base de données BDD qui contient un nombre prédéterminé de spectres de référence SR, c'est-à-dire de spectres d'absorption infrarouge de carcasses d'animaux mâles associés à des notes d'odeur qui ont été attribuées préalablement par des opérateurs après détection par le nez humain.

De plus, cet équipement informatique contient également une base de données avec un certain nombre de spectres aberrants SA. On entend par l'expression "spectre aberrant" un spectre qui ne respecte pas des paramètres prédéfinis. A titre d'exemple, ces paramètres peuvent consister en une anomalie sur le temps de contact entre la sonde et la carcasse, le positionnement de la sonde (si la sonde n'est pas mise en contact avec la carcasse, ou si la sonde est positionnée au mauvais endroit sur la carcasse), etc.

L'installation comporte par ailleurs des moyens de visualisation de l'information qui sera délivrée au final par l'installation, information relative à l'odeur attribuée à la carcasse.

Ces moyens de visualisation sont par exemple constitués par l'écran de l'ordinateur 4 précité.

Plus globalement, le procédé d'aide à la détection de carcasses odorantes conforme à l'invention comprend au moins les étapes suivantes qui consistent à :
a/ lecture de ladite information relative au sexe ;
b/ positionnement de ladite sonde relativement à ladite carcasse et activation de celle-ci pour mesurer ledit spectre ;
c/ vérification de la qualité du spectre mesuré, c'est à dire détermination de l'appartenance ou non aux spectres aberrants, avant de lui attribuer une note d'odeur obtenue ;
d/ visualisation de la note obtenue, en troisième lieu, par ledit équipement informatique ;
e/ affectation à ladite carcasse de ladite note.

Lorsque la sonde utilisée est une sonde de contact, on la positionne en surface de ladite carcasse, préférentiellement sur la partie découverte de l'échine.

En revanche, lorsque la sonde est une sonde de pénétration, on la positionne à une profondeur d'environ 5 cm par rapport à la surface de la carcasse, préférentiellement dans la région de l'échine.

On notera à cet égard que le gras du porc dans la région de l'échine est non homogène, car il est constitué de couches successives qui se différencient en profils d'acides gras. Dans ces conditions, il est indispensable de cibler correctement l'endroit ou la sonde doit être appliquée.

En tout état de cause, le principe à la base de l'invention est, pour chaque carcasse C, d'acquérir le spectre d'information infrarouge par le spectromètre relié à la sonde 3, de transférer ce spectre à un équipement informatique, en vue d'afficher un résultat relatif à la note d'odeur attribuée à ladite carcasse.

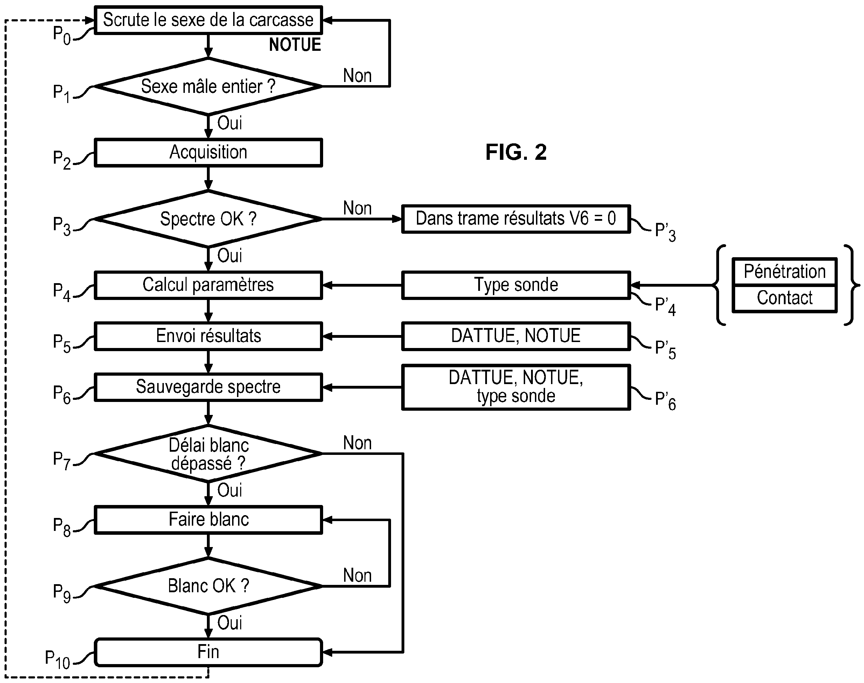

En se reportant à la figure 2, on peut illustrer ci-après le déroulement de ce processus.

Ainsi, dans une étape P₀, la cellule 2 est en attente de la détection d'une nouvelle carcasse C.

Cette même cellule 2 lit, à l'étape P₁, l'information relative au sexe et, préférentiellement le numéro de tuerie et le cas échéant celle concernant la date d'abattage.

Le système attend alors que le bouton d'actionnement de la sonde 3 soit activé. Ceci correspond au poste P₂ de la figure 2. Dans l'hypothèse où celui-ci est effectivement activé, on passe au poste P₃ d'acquisition du spectre via la sonde qui travaille en collaboration avec le spectromètre précité.

Cette opération est réalisée en mettant en contact la sonde 3 avec le gras de la carcasse C

Lorsque le spectre est enregistré (poste P₃), l'équipement informatique le compare à ceux présents dans la base de données BDD et vérifie si ce spectre est similaire à un spectre aberrant SA.

Pour ce faire, l'équipement informatique est par exemple configuré pour calculer l'aire sous le spectre, enregistrer sa forme et comparer ces données à celles de la "collection" de spectres aberrants.

Dans cette éventualité, une information relative à la mesure d'un spectre aberrant est affichée sur l'interface visuelle de l'ordinateur 4 et est stockée informatiquement (poste P'₃).

Dans l'hypothèse où ce spectre est correct, le système informatique récupère l'information relative au type de sonde (de contact ou de pénétration). Cela correspond au poste P'₄.

Ensuite, la note d'odeur prédite au poste P₄.

Concrètement, des algorithmes de prédiction sont développés par étalonnage des spectres de mesures infrarouge sur le gras des carcasses avec les notes des opérateurs obtenues sur les mêmes carcasses selon la méthode dite des "Nez Humains". L'étalonnage est obtenu préférentiellement par chimiométrie selon la méthode de régression des moindres carrés partiels (PLS).

Au poste P₅, le résultat (note) est affiché et, au poste P'₅, l'information "date et numéro de tuerie" est alors enregistrée et associée au résultat (note). Au poste P₆, on procède de même pour le spectre qui a été mesuré.

Dans une étape ultérieure, au poste P₇, le système détecte si un temps correct est utilisé pour un "ré-étalonnage" de l'appareil. Si ce délai est dépassé, on procède alors à un "blanc", c'est-à-dire une reconfiguration du spectromètre. Dans le cas contraire, on arrive directement à l'étape P₁₀, c'est-à-dire à la fin du processus, et on réitère alors ce processus en mettant en oeuvre le poste P₀.

La reconfiguration de l'appareil (ou étalonnage) peut être réalisée en prenant comme matériau de référence celui connu sous la marque "Spectralon".

Lorsqu'une carcasse C a été mesurée, elle est acheminée vers la chambre de stockage ou elle est regroupée avec les carcasses possédant la même note, en vue d'une affectation et une utilisation prédéterminées.

Parmi les avantages liés à l'installation et au procédé selon l'invention, on retiendra principalement les avantages suivants :
- Une prédiction fiable et rapide de la note d'odeur, puisque seulement 3 à 4 secondes sont nécessaires pour analyser chaque carcasse.
- Une méthode automatisée fonctionnant en ligne et pouvant communiquer avec un système informatique pour l'identification des carcasses analysées et la bonne affectation du résultat de l'analyse à la bonne carcasse.
- Une méthode industrielle qui respecte les cadences d'abattage avec environ 700 mesures par heure.
- Une méthode peu coûteuse par rapport à la méthode du « nez humain » car elle ne requiert qu'un seul opérateur.
- La facilité de préhension et la précision du geste de l'opérateur lors des prises de mesure, comparée notamment à la qualification des "Nez Humains jury expert".
- La fiabilité de la mesure (détection automatique des mesures aberrantes), sans aucune subjectivité de la note.
- La nettoyabilité et la robustesse de la sonde de mesure.

## Revendications

1. Installation (I) d'aide à la détection de carcasses (C) odorantes d'animaux abattus précédemment, **caractérisée par le fait qu'**elle comporte :
- au moins un support d'information, ce support d'information portant, pour chaque carcasse (C) d'animal, une information relative au moins au sexe dudit animal ;
- au moins un outil (2) de lecture et d'enregistrement de ladite information ;
- au moins une sonde (3) de lecture, reliée à un spectromètre infrarouge, ce dernier étant agencé pour mesurer le spectre d'absorption infrarouge desdites carcasses (C) ;
- au moins un équipement informatique (5) qui enregistre chaque spectre mesuré , équipement incorporant des algorithmes obtenus suite à une phase d'étalonnage avec une base de données contenant un nombre prédéterminé de "spectres de référence", c'est à dire de spectres d'absorption infra-rouge de carcasses associés à des notes d'odeur, ainsi qu' un certain nombre de "spectres aberrants", à savoir des spectres qui ne respectent pas des paramètres prédéfinis, lesdites notes d'odeur ayant été attribuées préalablement par des opérateurs après détection par le nez humain,
cet équipement informatique (5) étant configuré pour :
- en premier lieu, pour chaque carcasse testée (C) et seulement dans l'hypothèse où la carcasse testée provient d'un animal de sexe masculin, associer ladite information relative au sexe, lue par ledit outil de lecture (2) avec le spectre infrarouge mesuré associé,
- en deuxième lieu, comparer ce spectre infrarouge mesuré aux spectres de la base de données
- en troisième lieu et seulement si le spectre de la base de données le plus proche n'est pas considéré comme un spectre aberrant, affecter à ladite carcasse ladite note d'odeur associée;
- visualiser la note obtenue en troisième lieu.

2. Installation selon la revendication 1, **caractérisée par le fait que** ledit support d'information porte également une information relative au numéro de tuerie de l'animal.

3. Installation selon la revendication 1 ou 2, **caractérisée par le fait que** ladite sonde (3) est du type « par pénétration » ou du type « par contact », c'est à dire qu'elle est configurée pour réaliser une lecture par pénétration dans la carcasse, respectivement par contact avec la carcasse (C).

4. Installation selon la revendication 2, **caractérisée par le fait qu'**elle comprend des algorithmes configurés pour associer, à ladite carcasse, ladite note suivant le type de sonde (3).

5. Installation selon l'une des revendications précédentes, **caractérisée par le fait que** lesdits spectres sont des spectres d'absorption dans le proche infrarouge.

6. Installation selon l'une des revendications précédentes, **caractérisée par le fait que** ledit équipement informatique (5) est également configuré pour, en quatrième lieu, trier lesdites carcasses (C) en fonction de la note qui leur a été allouée.

7. Installation selon l'une des revendications précédentes, **caractérisée par le fait que** ledit équipement informatique comprend au moins un algorithme mathématique de prédiction de la note d'odeur.

8. Procédé d'aide à la détection de carcasses (C) odorantes d'animaux abattus précédemment, qui fait usage de l'installation (I) selon l'une des revendications précédentes, **caractérisé par le fait qu'**il comporte au moins les étapes suivantes :
a/ lecture de ladite information relative au moins au sexe ;
b/ positionnement de ladite sonde (3) relativement à ladite carcasse (C) et activation de celle-ci pour mesurer ledit spectre ;
c/ vérification de la qualité du spectre mesuré, c'est à dire détermination de l'appartenance ou non aux spectres aberrants, avant de lui attribuer une note d'odeur obtenue ;
d/ visualisation de la note obtenue, en troisième lieu, par ledit équipement informatique (5) ;
e/ affectation à ladite carcasse (C) de ladite note.

9. Procédé selon la revendication 8, **caractérisé par le fait que** lorsque la sonde (3) est une sonde de contact, on la positionne en surface de ladite carcasse (C), préférentiellement sur la partie découverte de l'échine.

10. Procédé selon la revendication 8, **caractérisé par le fait que** lorsque la sonde (3) est une sonde de pénétration, on la positionne à une profondeur d'environ 5 cm par rapport à la surface de la carcasse (C), préférentiellement dans la zone de l'échine.
